# EUROPEAN PATENT APPLICATION

(11) **EP 4 282 530 A1**
(43) Date of publication of application: **29.11.2023**
(21) Application number: 22820456.6
(22) Date of filing: 30.05.2022
(51) Int. Cl.: B01L 3/00, A61B 10/00, G01N 21/47

(54) **SAMPLE TRANSPORT MEDIUM VIAL**

(30) Priority: 10.06.2021 KR 20210075475
(71) Applicant: The Wave Talk, Inc., Daejeon 34051 (KR)
(72) Inventor: KIM, Young Dug, Seongnam-si, Gyeonggi-do 13597 (KR); CHO, Kyoung Man, Seoul 04410 (KR)
(74) Representative: Marks & Clerk LLP
(86) International application number: PCT/KR2022/007637
(87) International publication number: WO 2022/260328

(57) **Abstract**

The present invention relates to a sample transport medium vial, and may comprise: a sample collection part having an extraction part formed at the front end thereof, having a handle part formed in the middle thereof, and having, at the rear end thereof, a push button that can be pressed with the fingers; a medium accommodation tube in which a medium accommodation space capable of accommodating the medium is formed, and which is formed in a shape that encompasses the extraction part so that the extraction part of the sample collection part can be protected; and a one-touch-type elastic push device, which is provided at the handle part so that the protrusion length of the extraction part from the handle part can be extended to a first length or contracted to a second length when a collector presses the push button once or twice.

## Description

### Technical Field

The present invention relates to a sample transport medium vial, and more particularly, to a sample transport medium vial enabling the speckle pattern of a cultured medium to be checked such that contamination caused by bacteria or microorganisms can be confirmed.

### Background Art

In general, in order to obtain accurate results of microbiologic testing, it is necessary to send collected samples to a laboratory for immediate testing. When immediate testing is not possible, however, the collected samples have to be preserved or transported in an adequate environment.

In order to transport a sample, various instruments, such as a syringe, a sterilized plastic bottle, and the like, are used depending on the type (blood, pus, urine, stool, etc.) of the sample and a culture environment (anaerobic, aerotropic, virus, etc.) of transported bacteria. However, commercialized transport culture medium containers consisting of a plastic tube containing various bacterial culture media and a disposable sterile cotton swab, are usually used.

On one hand, in recent years, a speckle detection method has been developed and used to detect contamination caused by bacteria or microorganisms cultured in a medium in a non-contact manner, wherein by using laser light generated from a laser source, a speckle pattern of the culture medium due to multiple scattering by bacteria or microorganisms is photographed by a camera.

However, the speckle detection method cannot be applied to the conventional culture medium containers. That is, the aforementioned conventional transport culture medium container including a cotton swab, in which the cotton swab is immersed in a culture medium all the time, is not suitable for speckle pattern inspection, since the cotton swab and foreign matter separated from the cotton swab immersed in the culture medium for a long time may interfere with a primary optical axis of the laser light such that the laser light is obscured or blocked.

On the other hand, a conventional cotton swab has a constant protrusion length from a handle, and consequently, the range of sample collection and elasticity of the cotton swab are consistently fixed, which may be inconvenient in that a sample collector cannot adjust the length or elasticity of the cotton swab in consideration of the collection environment or his/her own physical characteristics when collecting a sample.

For example, when a sample is collected from deep inside the nose or the back of the throat, a longer cotton swap is required, and otherwise, a shorter cotton swab may be more manageable and offer a lower risk of contamination depending on the physical characteristics of a sample collector.

### Detailed Description of the Invention

### Technical problem

An object of the present invention is to provide a sample transport medium vial which allows a collector to easily use a cotton swab according to a collection environment or physical characteristics of the collector by making it possible to adjust the protrusion length of the cotton swab from a handle to be long or short with a simple push button mechanism during sample collection, enables smooth inspection of a speckle pattern of the culture medium by extending the protrusion length of the cotton swab to sufficiently mix the sample and the culture medium with each other in a state in which the cotton swab is immersed in the culture medium and then contracting the protrusion length of the cotton swab to raise the cotton swab such that the cotton swab does not obscure or block laser light, prevents the cotton swab from being immersed in the culture medium for a long time, thereby preventing foreign matter from being separated from the cotton swab and contaminating the culture medium, also allows the reliability and accuracy of speckle inspection to be greatly improved by partially or completely separating or blocking the cotton swab from the culture medium, and enables the calculation of a reference value of the speckle pattern by accommodating a control culture medium. However, the above object is illustrative only, and does not limit the scope of the present invention.

### Technical Solution

According to an aspect of the present invention to achieve the above object, there is provided a sample transport medium vial including: a sample collection part having an extraction part formed at the front end thereof, having a handle part formed in the middle thereof, and having, at the rear end thereof, a push button that can be pressed with the fingers; a medium accommodation tube in which a medium accommodation space capable of accommodating the medium is formed, and which is formed in a shape that encompasses the extraction part so that the extraction part of the sample collection part can be protected; and a one-touch-type elastic push device, which is provided at the handle part so that the protrusion length of the extraction part from the handle part can be extended to a first length or contracted to a second length when a collector presses the push button once or twice.

In addition, according to the present invention, the extraction part may include a cotton swab consisting of a cotton part and a rod part.

In addition, according to the present invention, the medium accommodation tube may include a body part detachably installed at the sample collection part; and a medium accommodation part formed below the body part and configured to accommodate therein the culture medium.

In addition, according to the present invention, the medium accommodation part may have a light entrance part formed of a light-transmitting material on one side thereof through which laser light generated from a laser source is input such that contamination caused by bacteria or microorganisms cultured in the culture medium can be detected in a non-contact manner, and a light exit part formed of a light-transmitting material on the other side thereof to allow a camera to photograph a speckle pattern of the culture medium generated due to multiple scattering of the laser light.

In addition, according to the present invention, the extraction part, when measuring the speckle pattern, may be raised up to a height that deviates from a primary optical axis connecting the light entrance part and the light exit part.

In addition, according to the present invention, at least one scattering protrusion may be formed on the light entrance part or the light exit part to promote multiple scattering.

In addition, according to the present invention, an optical coating layer capable of preventing fogging or growth of microorganisms may be formed on the outer surface of the light entrance part or light exit part.

In addition, according to the present invention, the one-touch-type elastic push device may include a generally short pipe-shaped cylinder body which is installed inside the handle part and in which a slide groove part including long grooves and short grooves formed alternately on the inner diameter surface thereof is formed; an overall rod-shaped button shaft part which is installed, at least in part, inside the cylinder part, is moved up and down by being connected to the push button, and has inclined projections formed along the outer diameter surface thereof; and an elevating rotary shaft part on which sliding projections that move up and down alternately along the long grooves and along the short grooves are formed.

In addition, according to the present invention, the one-touch-type elastic push device may further include an elevating non-rotary shaft part which has one end supporting the elevating rotary shaft part freely in rotation and the other end at which the collection part is installed; and an elastic spring which is installed between the elevating non-rotary shaft part and the handle part and has a restoring force that presses the elevating non-rotary shaft part toward the push button.

In addition, according to the present invention, the one-touch-type elastic push device may further include a sealing member which is installed between the elevating non-rotary shaft part and the handle part such that the medium accommodation space is decompressed when the elevating non-rotary shaft part moves up.

In addition, according to the present invention, the medium accommodating tube may further include a separation wall part which is formed to protrude in the inner diameter direction between the body part and the medium accommodation part and has an extraction part passage along which the extraction part moves up and down.

In addition, according to the present invention, the medium accommodation tube may further include a blocking device installed in the extraction part passage and configured to seal the medium accommodation part.

In addition, according to the present invention, the blocking device may include at least one of an aperture type, a valve type, a one-way stopper type, a thin film type, a sliding type, or combinations thereof.

In addition, according to the present invention, the medium accommodation tube may further include a separation device installed on the body part or the medium accommodation part to separate the body part and the medium accommodation part from each other.

Also, according to the present invention, the separation device may include at least one of a forced engagement type, a twist cap type, a magnetic engagement type, a hook assembly type, or combinations thereof.

In addition, according to the present invention, the sample transport medium vial may further include a control medium accommodation tube which is located near, and isolated from, the medium accommodation tube, and in which a control medium accommodation space capable of accommodating a control culture medium is formed.

In addition, according to the present invention, the control medium accommodation tube may have a light entrance part formed of a light-transmitting material on one side thereof through which laser light generated from a laser source is input and a light exit part formed of a light-transmitting material on the other side thereof to allow a camera to photograph a speckle pattern of the control culture medium generated due to multiple scattering of the laser light.

According to an aspect of the present invention to achieve the above object, there is provided a sample transport medium vial including a sample collection part having an extraction part formed at the front end thereof; a medium accommodation tube in which a medium accommodation space capable of accommodating the medium is formed, and which is formed in a shape that encompasses the extraction part so that the extraction part of the sample collection part can be protected; and a control medium accommodation tube which is located near, and isolated from, the medium accommodation tube, and in which a control medium accommodation space capable of accommodating a control culture medium is formed.

### Effect of the Invention

According to an embodiment of the present invention as described above, a collector is allowed to adjust the protrusion length of a cotton swab from a handle to be long or short with a simple push button mechanism during sample collection, so that the collector can easily use the cotton swab according to the collection environment or his/her own physical characteristics, smooth inspection of a speckle pattern of the culture medium may be enabled by extending the protrusion length of the cotton swab to sufficiently mix the sample and the culture medium with each other in a state in which the cotton swab is immersed in the culture medium and then contracting the protrusion length of the cotton swab to raise the cotton swab such that the cotton swab does not obscure or block laser light, the cotton swab may be prevented from being immersed in the culture medium for a long time, thereby preventing foreign matter from being separated from the cotton swab and contaminating the culture medium, the reliability and accuracy of speckle inspection may be greatly improved by partially or completely separating or blocking the cotton swab from the culture medium, and a reference value of the speckle pattern may be calculated by accommodating a control culture medium. However, the above effects do not limit the scope of the present invention.

### Brief Description of Drawings

FIG. 1 is a perspective view showing a sample transport medium vial according to some embodiments of the present invention.
FIG. 2 is an exploded perspective view showing the sample transport medium vial of FIG. 1.
FIG. 3 is an exploded cross-sectional view showing the sample transport medium vial of FIG. 2.
FIG. 4 is a cross-sectional view showing a state in which a cotton swab is lowered in the sample transport medium vial of FIG. 1.
FIG. 5 is a cross-sectional view showing a primary button pressing state of the sample transport medium vial of FIG. 4.
FIG. 6 is a cross-sectional view showing a state in which a cotton swab is raised in the sample transport medium vial of FIG. 5.
FIG. 7 is a cross-sectional view showing a secondary button pressing state of the sample transport medium vial of FIG. 4.
FIG. 8 is an enlarged cross-sectional view showing a separation wall part of a sample transport medium vial according to some other embodiments of the present invention.
FIG. 9 is an enlarged cross-sectional view showing a blocking device of a sample transport medium vial according to some other embodiments of the present invention.
FIG. 10 illustrates views of various embodiments of the blocking device of the sample transport medium vial of FIG. 9.
FIG. 11 is an enlarged cross-sectional view showing a separation device of a sample transport medium vial according to some other embodiments of the present invention.
FIG. 12 illustrates views of various embodiments of the separation device of the sample transport medium vial of FIG. 11.
FIG. 13 is a cross-sectional view showing a sample transport medium vial according to some other embodiments of the present invention.

### Mode for Invention

The present invention will now be described in detail in connection with preferred embodiments with reference to the accompanying drawings. The present invention may, however, be embodied in many different forms and should not be construed as limited to the exemplary embodiments set forth herein. Rather, these embodiments are provided so that this disclosure will be thorough and complete, and will fully convey the scope of the inventive concept to one of ordinary skill in the art. Sizes of components in the drawings may be exaggerated for convenience of explanation.

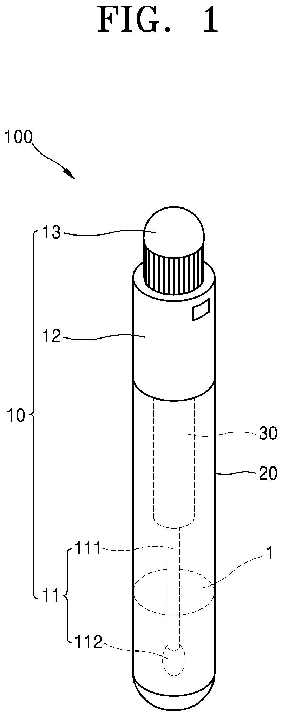

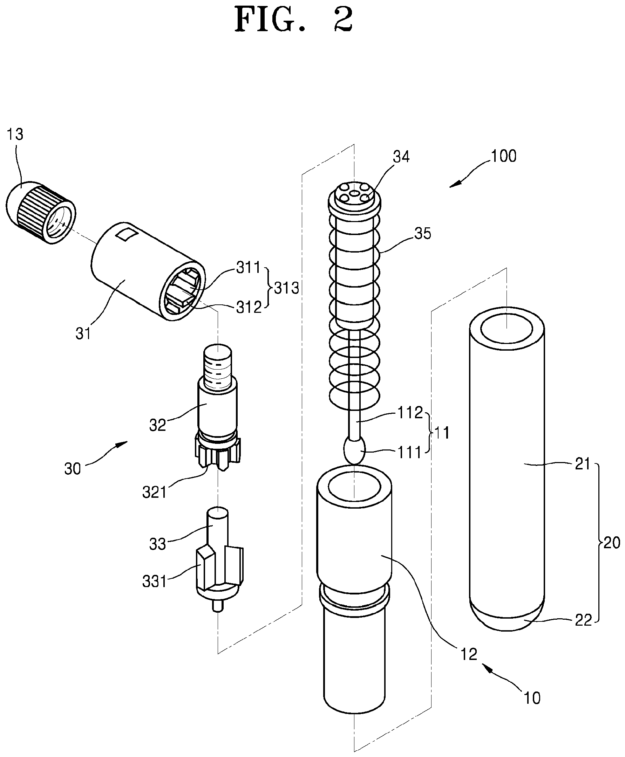

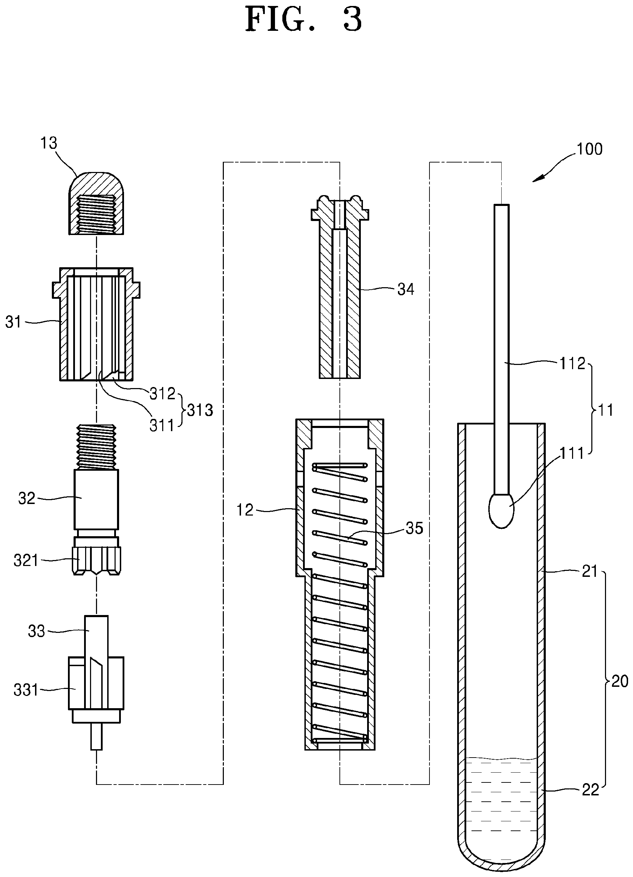

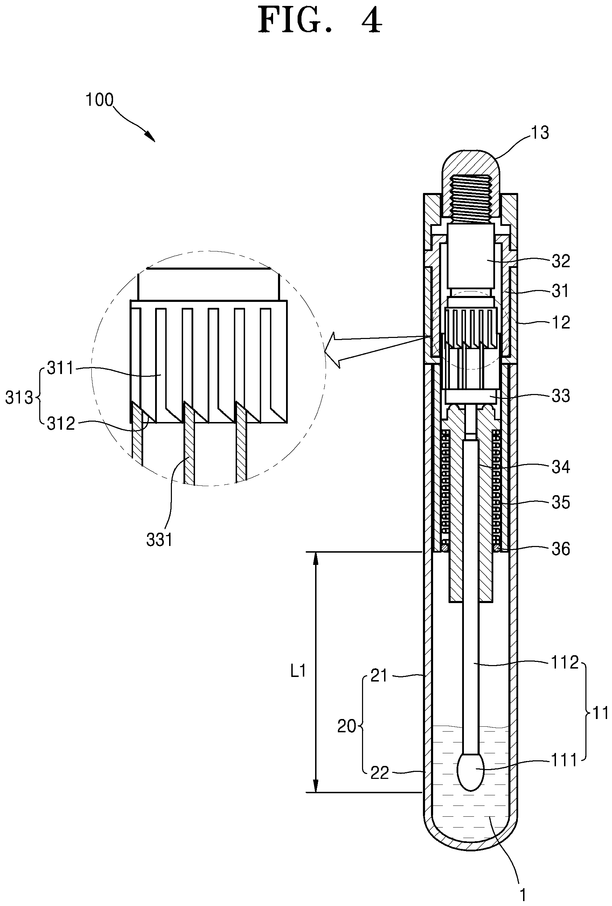

FIG. 1 is a perspective view showing a sample transport medium vial 100 according to some embodiments of the present invention, FIG. 2 is an exploded perspective view showing the sample transport medium vial 100 of FIG. 1, FIG. 3 is an exploded cross-sectional view showing the sample transport medium vial 100 of FIG. 2, and FIG. 4 is a cross-sectional view showing a state in which a cotton swab is lowered in the sample transport medium vial 100 of FIG. 1.

First, as shown in FIGS. 1 to 4, the sample transport medium vial 100 according to some embodiment may largely include a sample collection part 10, a medium accommodation tube 20, and a one-touch-type elastic push device 30.

Here, the sample collection part 10 may be detachably coupled to the medium accommodation tube 20, and the one-touch-type elastic push device 30 may be installed at the sample collection part 10 so that a collector can separate the sample collection part 10 from the medium accommodation tube 20 and uses it even when collecting a sample.

For example, as shown in FIGS. 1 to 4, the sample collection part 10 may have an extraction part 11 formed at the front end thereof, have a handle part 12 formed in the middle thereof, and have, at the rear end thereof, a push button 13 that can be pressed with a finger.

More specifically, for example, the extraction part 11 may include a cotton swab consisting of a cotton part 11 formed of sterilized cotton and a rod part 112 in the form of a stick. However, the extraction part 11 is not limited to the aforementioned cotton swab, and extraction parts having various shapes, such as collection plates, collection brush, and collection protrusions, may all be applicable.

In addition, for example, as shown in FIGS. 1 to 4, the medium accommodation tube 20 may be a cylindrical-shaped structure in which a medium accommodation space capable of accommodating therein a culture medium 1 in liquid form, solid state, or gel form is formed, and may be formed in a shape that encompasses the extraction part 11 so that the extraction part 11 of the sample collection part 10 can be protected.

However, the medium accommodation tube 20 is not necessarily limited to the drawing, and may be applied in a variety of shapes, such as an elliptical or polygonal pipe shape or various geometric shapes, in addition to the illustrated circular cylinder shape.

More specifically, for example, the medium accommodation tube 20 may include a body part 21 detachably installed at the sample collection part 10 and a medium accommodation part 22 formed below the body part 21 and configured to accommodate therein the culture medium 1.

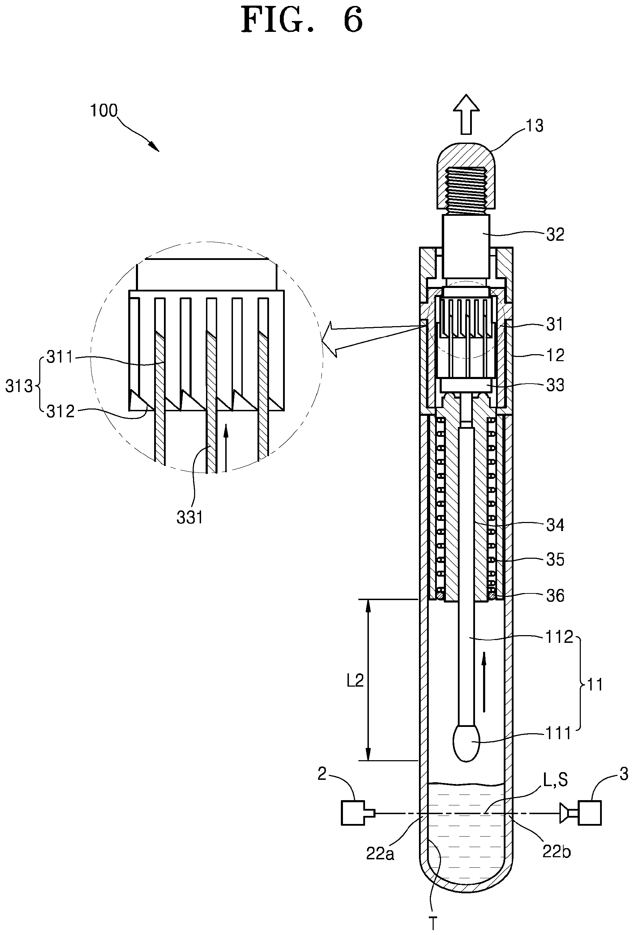

Meanwhile, for example, as shown in FIGS. 1 to 4, the one-touch-type elastic push device 30 may be a device that is installed at the handle part 12 so that the protrusion length of the extraction part 11 from the handle part 12 can be extended to a first length L1 shown in FIG. 4 or contracted to a second length L2 shown in FIG. 6 when the collector presses the push button 13 once or twice.

More specifically, for example, as shown in FIGS. 1 to 4, the one-touch-type elastic push device 30 may include a generally short pipe-shaped cylinder body 31 which is installed inside the handle part 12 and in which a slide groove part 313 including long grooves 311 and short grooves 312 formed on the inner diameter surface thereof is formed; an overall rod-shaped button shaft part 32 which is installed, at least in part, inside the cylinder part 31, is moved up and down by being connected to the push button 13, and has inclined projections 321 formed along the outer diameter surface thereof, and an elevating rotary shaft part 33 on which sliding projections 331 that move up and down alternately along the long grooves 311 and along the short grooves 312 are formed.

In addition, for example, the one-touch-type elastic push device 30 may further include an elevating non-rotary shaft part 34 which has one end supporting the elevating rotary shaft part 33 freely in rotation and the other end at which the collection part 11 is installed, an elastic spring 35 which is installed between the elevating non-rotary shaft part 34 and the handle part 12 and has a restoring force that presses the elevating non-rotary shaft part 34 toward the push button 13, and a sealing member 36 which is installed between the elevating non-rotary shaft part 34 and the handle part 12 such that the medium accommodation space is decompressed when the elevating non-rotary shaft part 34 moves up.

Thus, according to the one-touch-type elastic push device 30, when the collector presses the push button 13, the button shaft part 32 may rotate the elevating rotary shaft part 33 at multiple angles while being connected to the push button 13 and descending, the elevating rotary shaft part 33 may move up and down alternately along the long grooves 311 of the slide groove part 313 of the cylinder part 31 and along the short grooves 312 while overcoming the restoring force of the elastic spring 35, and the elevating non-rotary shaft part 34 may be moved up and down in a non-rotating state so that the cotton swab can be operated in two modes, one in which the cotton swab is lowered and one in which it is raised.

At this time, a gap between the handle part 12 to which the sealing member 36 is fixed and the moving elevating non-rotary shaft part 34 may be sealed so that the medium accommodation space is decompressed when the elevating non-rotary shaft part 34 moves up, thereby minimizing the generation of air bubbles during speckle inspection, and contamination caused by external air or foreign matter that may penetrates into the gap between the handle part 12 and the elevating non-rotary shaft part 34 can be prevented.

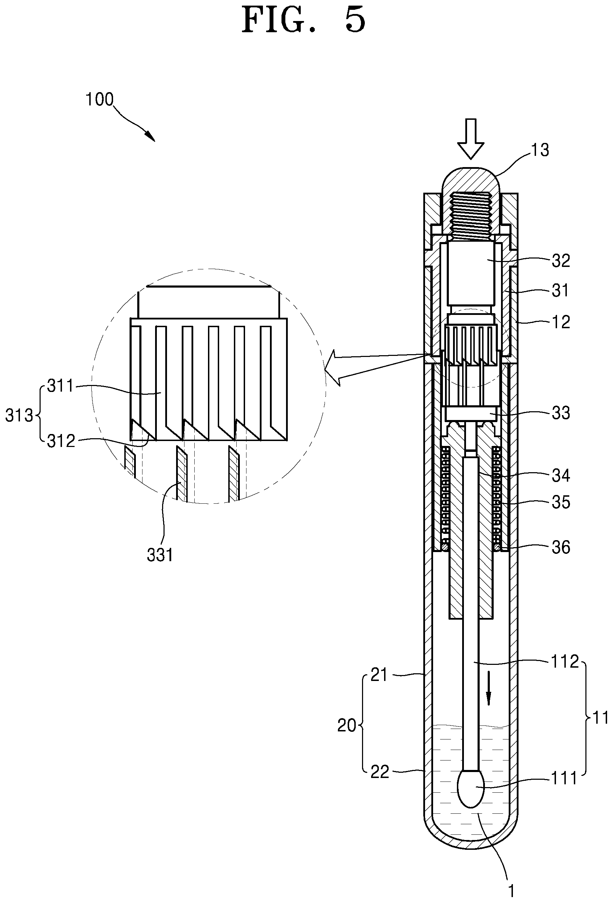

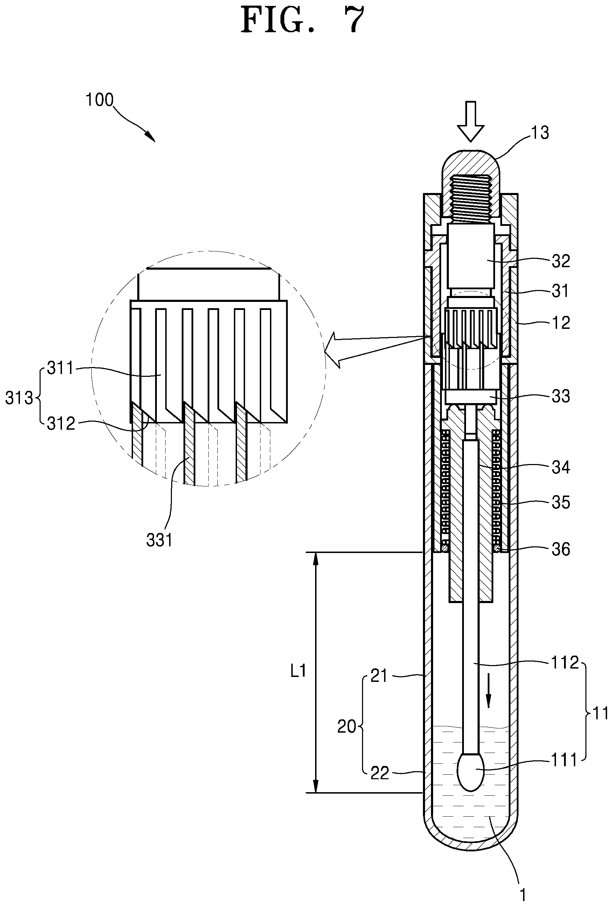

FIG. 4 is a cross-sectional view showing a state in which the cotton swab is lowered in the sample transport medium vial 100 of FIG. 1, FIG. 5 is a cross-sectional view showing a primary button pressing state of the sample transport medium vial 100 of FIG. 4, FIG. 6 is a cross-sectional view showing a state in which a cotton swab is raised in the sample transport medium vial 100 of FIG. 5, and FIG. 7 is a cross-sectional view showing a secondary button pressing state of the sample transport medium vial 100 of FIG. 4.

As shown in FIGS. 4 to 7, the operation process of the sample transport medium vial 100 according to some embodiments of the present invention is as follow. First, as shown in FIG. 4, a collector or a user may couple the sample collection part 10 to the medium accommodation tube 20 in a state in which the protrusion length of the extraction part 11 from the handle part 12 is extended to the first length L 1.

At this time, as shown in the enlarged portion of FIG. 4, the slide groove part 313 of the elevating rotary shaft part 33 may be caught on the short grooves 312 of the slide groove part 313 and remain in the lowered state.

Accordingly, the cotton part 111 is immersed in the culture medium 1 so that a portion of the sample collected on the cotton part 111 can be sufficiently mixed with the culture medium 1.

Subsequently, as shown in FIG. 5, when the collector or the user presses the push button 13 once, as shown in the enlarged portion of FIG. 5, the slide groove part 313 of the elevating rotary shaft part 33 may be dislodged from the short grooves 313 of the slide groove part 313 and moved along inclined surfaces in the direction of the neighboring long grooves 311 of the slide groove part 313 while rotating at multiple angles by the inclined projections 321 of the button shaft part 32.

Subsequently, as shown in FIG. 6, when the collector or the user releases the push button 13, as shown in the enlarged portion of FIG. 6, the slide groove part 313 of the elevating rotary shaft part 33 may be moved up along the long grooves 311 of the slide groove part 313 by the restoring force of the elastic spring 35, so that the protrusion length of the extraction part 11 from the handle part 12 can be contracted to the second length L2.

Therefore, the culture medium 1 may be cultured for a sufficient amount of time (several minutes to several hours) for speckle inspection of the bacteria or microorganisms contained in the sample in a state in which the influence of the cotton swab is excluded.

At this time, as shown in FIG. 6, the medium accommodation part 22 may have a light entrance part 22a formed of a light-transmitting material on one side thereof through which laser light L generated from a laser source 2 is input such that contamination caused by the bacteria or microorganisms cultured in the culture medium 1 can be detected in a non-contact manner, and a light exit part 22b formed of a light-transmitting material on the other side thereof to allow a camera 3 to photograph a speckle pattern of the culture medium 1 generated due to multiple scattering of the laser light L. When measuring the speckle pattern, the extraction part 11 is raised up to a height that deviates from the primary optical axis S connecting the light entrance part 22a and the light exit part 22b, and thus the speckle inspection may be smoothly performed.

Here, at least one scattering protrusion T may be formed on the light entrance part 22a or the light exit part 22b to promote multiple scattering, and an optical coating layer C capable of preventing fogging or growth of microorganisms may be formed on the outer surface of the light entrance part or light exit part to reduce the possibility of a change in optical characteristics due to fogging or growth of microorganisms.

More specifically, for example, the sample transport medium vial 100 of the present invention may be applicable to a speckle inspection device, for example, the speckle inspection device may use a non-contact speckle sensor configured to emit the laser light L to the culture medium 1 and detect a speckle pattern generated due to multiple scattering for the culture medium 1, and the principle of a chaotic wave sensor may be applied thereto.

For example, according to the principle of a chaotic wave sensor, in the case of a material with a homogeneous internal refractive index, such as glass, refraction occurs in a certain direction when coherent light is irradiated thereto.

However, when coherent light, such as laser light, is irradiated onto an object having a heterogeneous internal refractive index or formed of fine refraction or scattering protrusions, very complex multiple scattering occurs inside the material.

Some of the light rays that have been scattered through complicated paths due to multiple scattering pass through the culture medium 1, which is a test target object. Light rays passing through multiple points in the test target object generate constructive interference or destructive interference, and the constructive/destructive interference of the light rays generates grain patterns (speckles).

The light rays scattered along the complicated paths are referred to as "chaotic waves," and the chaotic waves may be detected through coherent light speckles, and in the case where the coherent light is laser light, the coherent light speckles may be detected through laser speckles.

When a stable medium is irradiated with coherent light, i.e., when a stable medium, in which an internal component does not move, is irradiated with coherent light (e.g., laser light), a stable speckle pattern without a variation may be observed.

However, when an unstable medium having an internal component that is moving, such as bacteria, is included therein, the speckle pattern changes.

That is, due to microscopic biological activities of microorganisms (e.g., intracellular movement, movement of microorganisms, etc.), an optical path may slightly change over time. Since the speckle pattern is generated by interference of light, a fine change in the optical path may cause variation in the speckle pattern. Accordingly, when a temporal variation in the speckle pattern is measured, the biological activities of microorganisms may be rapidly measured. As such, when the variation in the speckle pattern over time is measured, the presence or absence of microorganisms and concentration thereof may be identified, and furthermore, kinds of microorganisms may also be identified.

The test target object described in this specification is the cultured culture medium 1, and a configuration for measuring the variation in the speckle pattern of the culture medium 1 over time may be defined as a chaotic wave sensor.

Here, the chaotic wave sensor may be configured in various types, such as a reflective type and a transmissive type, and an optical system may be configured in a packaging type.

In addition, the laser source 2 may use laser light L having good coherence as a light source. However, in addition to the laser light source, a light source having improved coherence by including a filter that passes only a wavelength of a specific band or specific wavelength in a general illumination source may be used. Alternatively, the measurement may be performed using a wavelength (e.g., infrared, ultraviolet, etc.) outside the visible light range.

Also, the camera 3 that is a photographing device for photographing an image, and various image sensors may be applied thereto. When the culture medium 1 is irradiated with coherent light, a coherent light speckle may be formed by multiple scattering. If viruses, bacteria, microorganisms, etc. are present in the culture medium 1, the presence and absence of bacteria and microorganisms and concentration thereof in the test target object may be rapidly determined based on a pattern of the coherent light speckles that vary over time.

For example, as the test target object is irradiated with coherent light every reference time at regular intervals, a coherent light speckle may be formed in the test target object, and as the test target object in which multiple scattering occurs is photographed using the camera or the like, a coherent light speckle image of the formed coherent light speckle may be generated. In this case, a camera including a two-dimensional image sensor or a one-dimensional optical sensor may be used to measure a speckle pattern of a plurality of generated images. For example, a camera equipped with an imaging device, such as a charge-coupled device (CCD) may be used as a measurement unit.

Therefore, whether bacteria and microorganisms are present in the culture medium 1 may be determined from the photographed coherent light speckle images in a non-contact manner by checking whether the coherent light speckles are changed in pattern over time. For example, if there is no activity in the culture medium 1, coherent light speckles appear with a constant interference pattern over time. That is, if there is no activity, a constant interference pattern of the coherent light speckles may be found in the images of the coherent light speckles measured every reference time. As such, when the coherent light speckle images show no, or very little, variation of the interference pattern over time, it may be determined that bacteria and microorganisms are absent in the culture medium 1.

On the other hand, when the pattern of the coherent light speckles changes, it may be determined that bacteria and microorganisms are present in the culture medium 1. That is, when bacteria and microorganisms are present in the culture medium 1, the bacteria and microorganisms may multiply over time, and the bacteria and microorganisms may continuously move. This movement of the bacteria or microorganisms may cause the continuous change of the pattern of laser speckles over time. Accordingly, when the pattern of the coherent light speckles has changed by a degree greater than or equal to a predetermined error range in the coherent light speckle images measured every reference time, it may be determined that bacteria and microorganisms are present in the culture medium 1.

In this case, the degree of change in the pattern of the coherent light speckles may be determined according to the concentration of bacteria and microorganisms. Accordingly, the concentration of the bacteria and microorganisms may be measured through temporal correlation analysis. For example, a standard deviation of light intensity of coherent light speckles may be used to measure the degree of change in the pattern of the coherent light speckles.

Subsequently, as shown in FIG. 7, the collector or the user may click the push button 13 twice at any time as needed such that the slide groove part 313 of the elevating rotary shaft part 33 is caught on the short grooves 312 of the slide groove part 313 and remains in the lowered state. Also, the collector or the user may separate the medium accommodation tube 20 from the sample collection part 10 and collect a sample only with the sample collection part 10 by adjusting the length of the extraction part 11 to be long or short by clicking the push button 13 multiple times.

Therefore, the collector is allowed to adjust the protrusion length of a cotton swab from a handle part 12 to be long L1 or short L2 with a simple push button mechanism during sample collection, so that the collector can easily use the cotton swab according to the collection environment or his/her own physical characteristics, smooth inspection of a speckle pattern of the culture medium 1 may be enabled by extending the protrusion length of the cotton swab to sufficiently mix the sample and the culture medium with each other in a state in which the cotton swab is immersed in the culture medium 1 and then contracting the protrusion length of the cotton swab to raise the cotton swab such that the cotton swab does not obscure or block the laser light, the cotton swab may be prevented from being immersed in the culture medium for a long time, thereby preventing foreign matter from being separated from the cotton swab and contaminating the culture medium.

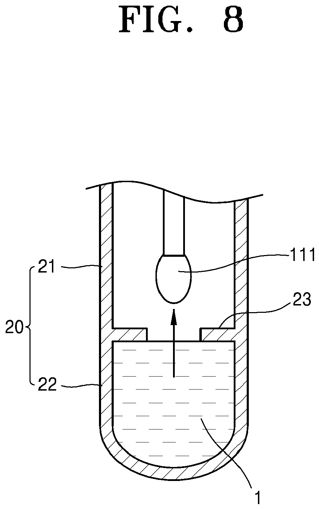

FIG. 8 is an enlarged cross-sectional view showing a separation wall part 23 of a sample transport medium vial according to some other embodiments of the present invention.

As shown in FIG. 8, the medium accommodating tube 20 may further include a separation wall part 23 which is formed to protrude in the inner diameter direction between the body part 21 and the medium accommodation part 22 and has an extraction part passage 23a along which the extraction part 11 moves up and down.

Therefore, when the separation wall part 23 is used, the culture medium 1 may be substantially separated from the cotton part 111 while partially blocking the adverse effects of the cotton part 111 on the culture medium 1, and the culture medium 1 accommodated in the medium accommodation part 22 may be partially prevented from leaking to the body part 21.

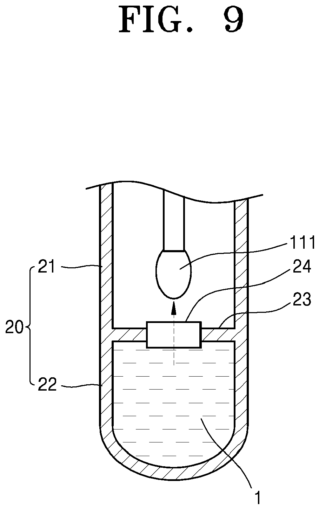

FIG. 9 is an enlarged cross-sectional view showing a blocking device 24 of a sample transport medium vial according to some other embodiments of the present invention.

As shown in FIG. 9, the medium accommodation tube 20 may further include the blocking device 24 installed in the extraction part passage 23a along with the separation wall part 23 and configured to seal the medium accommodation part 22.

Here, the blocking device 24 is capable of sealing the medium accommodation part 22 after passing the cotton swab, and various shapes and types of blocking devices may all be applied thereto.

Therefore, when the blocking device 24 is used, the culture medium 1 may be completely separated from the cotton part 111 while entirely blocking the adverse effects of the cotton part 111 on the culture medium 1, and the culture medium 1 accommodated in the medium accommodation part 22 may be entirely prevented from leaking to the body part 21.

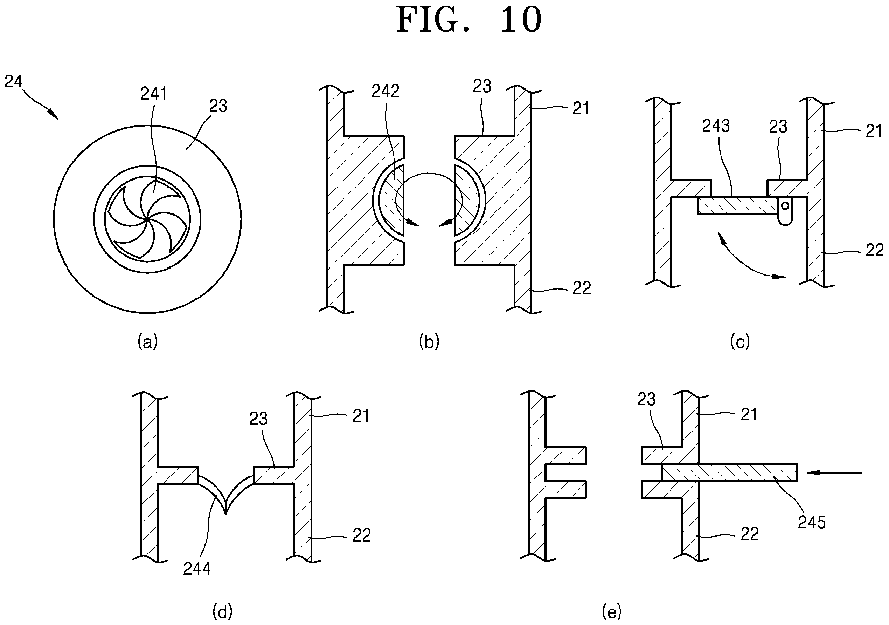

FIG. 10 illustrates views of various embodiments of the blocking device 24 of the sample transport medium vial of FIG. 9.

More specifically, for example, the blocking device 24 may include one or more of the following: an aperture type 241 capable of opening and closing a passage, as shown in (a) of FIG. 10, a valve type 242 capable of rotating a valve body, as shown in (b) of FIG. 10, a one-way stopper type 243 that rotates in one direction by an elastic spring, as shown in (c) of FIG. 10, a thin film type 244 similar to the heart valve or the like, as shown in (d) of FIG. 10, a sliding type 245 that slides like a sliding door, as shown in (e) of FIG. 10, and combinations thereof.

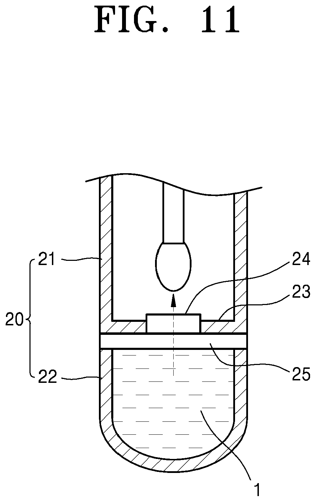

FIG. 11 is an enlarged cross-sectional view showing a separation device 25 of a sample transport medium vial according to some other embodiments of the present invention.

As shown in FIG. 11, the medium accommodation tube 20 may further include a separation device 25 installed on the body part 21 or the medium accommodation part 22 to separate the body part 21 and the medium accommodation part 22 from each other.

Therefore, the body part 21 and the medium accommodation part 22 may be detachably formed and completely separated by using the separation device 25.

Here as, the separation device 25, various shapes and types of separation devices that allow the body part 21 and the medium accommodation part 22 to be detachably formed may all be applied.

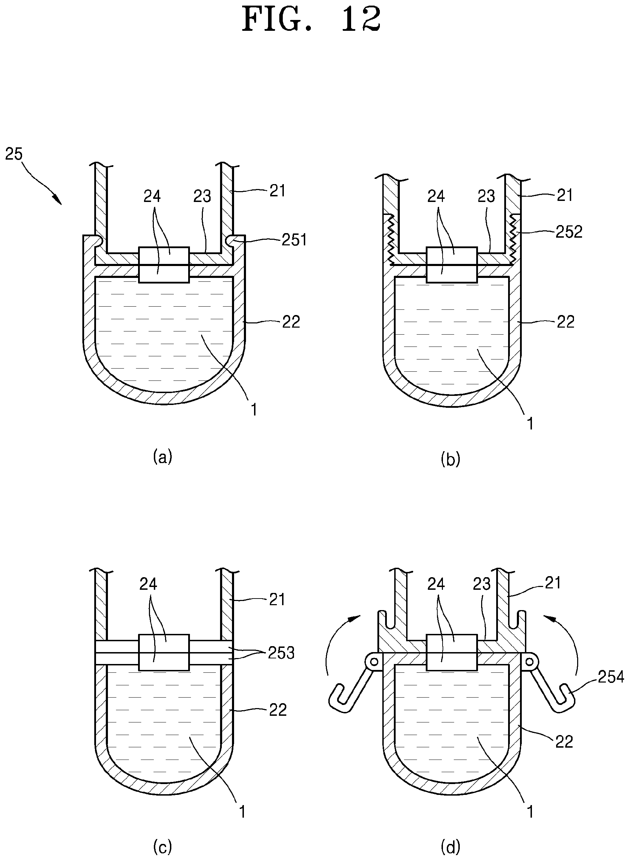

FIG. 12 illustrates views of various embodiments of the separation device 25 of the sample transport medium vial of FIG. 11.

More specifically, for example, the separation device 25 may include one or more of the following: a forced engagement type 251 including projections and groove parts engaged with each other, as shown in (a) of FIG. 12, a twist cap type 252 using a male thread part and a female thread part, as shown in (b) of FIG. 12, a magnetic engagement type 253 using a magnetic force of a permanent magnet or a magnetic body, as shown in (c) of FIG. 12, a hook assembly type 254 using a hook latch, as shown in (d) of FIG. 12, and combinations thereof.

Therefore, it is possible to enable smooth inspection of a speckle pattern of the culture medium by raising the cotton swab, as well as to prevent the cotton swab from being immersed in the culture medium for a long time and thus prevent foreign matter from being separated from the cotton swab and contaminating the culture medium. In addition, it is possible to greatly improve the reliability and accuracy of the speckle inspection by partially or completely separating or blocking the cotton swab from the culture medium.

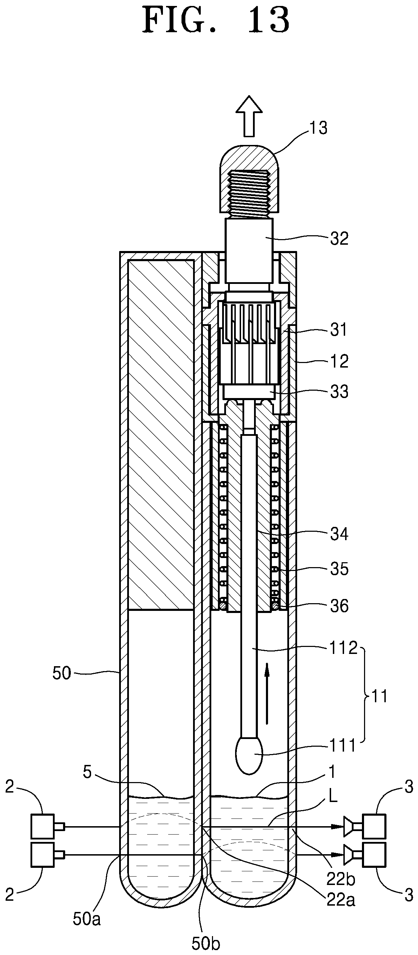

FIG. 13 is a cross-sectional view showing a sample transport medium vial 200 according to some other embodiments of the present invention.

As shown in FIG. 13, the sample transport medium vial 200 according to some other embodiments of the present invention may further include a control medium accommodation tube 50 which is located near, and isolated from, the medium accommodation tube 20 and in which a control medium accommodation space capable of accommodating a control culture medium 5 is formed.<

Here, the control medium accommodation tube 50 may have a light entrance part 50a formed of a light-transmitting material on one side thereof through which laser light L generated from a laser source 2 is input and a light exit part 50b formed of a light-transmitting material on the other side thereof to allow a camera 3 to photograph a speckle pattern of the control culture medium 5 generated due to multiple scattering of the laser light L by comparing the speckle pattern of the control culture medium 5 with the speckle pattern of the culture medium 1.

The control culture medium 5 is isolated from the extraction part 11, and may have the same components as those of the pure, initial culture medium 1 unaffected by the extraction part 11 for comparison of the speckle patterns.

Therefore, the laser source 2 may primarily emit the laser light L to the control culture medium 5 accommodated in the control medium accommodation tube 50, and a speckle pattern of the control culture medium 5 may be measured with the camera 3, such that a reference value is calculated. In this regard, the laser source 2 may secondarily emit the laser light L to the culture medium 1 contaminated by the extraction part 11 and accommodated in the medium accommodation tube 20, and the speckle pattern of the culture medium 1 may be measured with the camera 3 such that contamination caused by the cultured bacteria or microorganisms may be more accurately calculated based on the reference value.

Here, one set of the laser source 2 and the camera 3 may be moved and measure separately the medium accommodation tube 20 and the control medium accommodation tube 50, and a plurality of sets may be fixedly installed and simultaneously measure each of the medium accommodation tube 20 and the control medium accommodation tube 50.

On the other hand, in some other embodiments of the present invention, only the extraction part 11 may be installed in the medium accommodation tube 20 in a state where the medium accommodation tube 20 and the control medium accommodation tube 50 are installed, and the one-touch-type elastic push device 30 may be omitted.

Although the present invention has been described in connection with the exemplary embodiment shown in the drawings, it is only illustrative. It will be understood to those skilled in the art that various modifications and equivalents can be made without departing from the scope and spirit of the invention. Therefore, the scope of the present invention should be defined only by the appended claims.

## Claims

1. A sample transport medium vial comprising:
a sample collection part having an extraction part formed at the front end thereof, having a handle part formed in the middle thereof, and having, at the rear end thereof, a push button that can be pressed with the fingers;
a medium accommodation tube in which a medium accommodation space capable of accommodating the medium is formed, and which is formed in a shape that encompasses the extraction part so that the extraction part of the sample collection part can be protected; and
a one-touch-type elastic push device, which is provided at the handle part so that the protrusion length of the extraction part from the handle part can be extended to a first length or contracted to a second length when a collector presses the push button once or twice.

2. The sample transport medium vial of claim 1, wherein the extraction part comprises a cotton swab consisting of a cotton part and a rod part.

3. The sample transport medium vial of claim 1, wherein the medium accommodation tube comprises
a body part detachably installed at the sample collection part; and
a medium accommodation part formed below the body part and configured to accommodate therein the culture medium.

4. The sample transport medium vial of claim 3, wherein the medium accommodation part has a light entrance part formed of a light-transmitting material on one side thereof through which laser light generated from a laser source is input such that contamination caused by bacteria or microorganisms cultured in the culture medium can be detected in a non-contact manner, and a light exit part formed of a light-transmitting material on the other side thereof to allow a camera to photograph a speckle pattern of the culture medium generated due to multiple scattering of the laser light.

5. The sample transport medium vial of claim 4, wherein the extraction part, when measuring the speckle pattern, is raised up to a height that deviates from a primary optical axis connecting the light entrance part and the light exit part.

6. The sample transport medium vial of claim 4, wherein at least one scattering protrusion is formed on the light entrance part or the light exit part to promote multiple scattering.

7. The sample transport medium vial of claim 4, wherein an optical coating layer capable of preventing fogging or growth of microorganisms is formed on the outer surface of the light entrance part or light exit part.

8. The sample transport medium vial of claim 1, wherein the one-touch-type elastic push device comprises a generally short pipe-shaped cylinder body which is installed inside the handle part and in which a slide groove part including long grooves and short grooves formed alternately on the inner diameter surface thereof is formed;
an overall rod-shaped button shaft part which is installed, at least in part, inside the cylinder part, is moved up and down by being connected to the push button, and has inclined projections formed along the outer diameter surface thereof; and
an elevating rotary shaft part on which sliding projections that move up and down alternately along the long grooves and along the short grooves are formed.

9. The sample transport medium vial of claim 8, wherein the one-touch-type elastic push device further comprises
an elevating non-rotary shaft part which has one end supporting the elevating rotary shaft part freely in rotation and the other end at which the collection part is installed; and
an elastic spring which is installed between the elevating non-rotary shaft part and the handle part and has a restoring force that presses the elevating non-rotary shaft part toward the push button.

10. The sample transport medium vial of claim 9, wherein the one-touch-type elastic push device further comprises a sealing member which is installed between the elevating non-rotary shaft part and the handle part such that the medium accommodation space is decompressed when the elevating non-rotary shaft part moves up.

11. The sample transport medium vial of claim 3, wherein the medium accommodating tube further comprises a separation wall part which is formed to protrude in the inner diameter direction between the body part and the medium accommodation part and has an extraction part passage along which the extraction part moves up and down.

12. The sample transport medium vial of claim 11, wherein the medium accommodation tube further comprises a blocking device installed in the extraction part passage and configured to seal the medium accommodation part.

13. The sample transport medium vial of claim 12, wherein the blocking device comprises at least one of an aperture type, a valve type, a one-way stopper type, a thin film type, a sliding type, or combinations thereof.

14. The sample transport medium vial of claim 11, wherein the medium accommodation tube further comprises a separation device installed on the body part or the medium accommodation part to separate the body part and the medium accommodation part from each other.

15. The sample transport medium vial of claim 14, wherein the separation device may include at least one of a forced engagement type, a twist cap type, a magnetic engagement type, a hook assembly type, or combinations thereof.

16. The sample transport medium vial of claim 1, wherein the sample transport medium vial further comprises a control medium accommodation tube which is located near, and isolated from, the medium accommodation tube, and in which a control medium accommodation space capable of accommodating a control culture medium is formed.

17. The sample transport medium vial of claim 16, wherein the control medium accommodation tube has a light entrance part formed of a light-transmitting material on one side thereof through which laser light generated from a laser source is input and a light exit part formed of a light-transmitting material on the other side thereof to allow a camera to photograph a speckle pattern of the control culture medium generated due to multiple scattering of the laser light.

18. A sample transport medium vial comprising:
a sample collection part having an extraction part formed at the front end thereof;
a medium accommodation tube in which a medium accommodation space capable of accommodating the medium is formed, and which is formed in a shape that encompasses the extraction part so that the extraction part of the sample collection part can be protected; and
a control medium accommodation tube which is located near, and isolated from, the medium accommodation tube, and in which a control medium accommodation space capable of accommodating a control culture medium is formed.
